# EUROPEAN PATENT APPLICATION

(11) **EP 4 729 547 A1**
(43) Date of publication of application: **22.04.2026**
(21) Application number: 24823456.9
(22) Date of filing: 14.06.2024
(51) Int. Cl.: C07K 19/00, C12N 9/10, C12N 15/09, C12N 15/63

(54) **ZINC FINGER PROTEIN, ZINC FINGER NUCLEASE, VECTOR, AND GENOME EDITING METHOD USING SAID SUBSTANCES**

(30) Priority: 16.06.2023 JP 2023098848
(71) Applicant: VCGT Inc., Kobe-shi, Hyogo, 650-0047 (JP); Hiroshima University, Higashi-Hiroshima-shi Hiroshima 739-8511 (JP)
(72) Inventor: ONISHI Akishi, Kobe-shi, Hyogo 650-0047 (JP); YASUDA Kazushi, Kobe-shi, Hyogo 650-0047 (JP); ISHIMARU Aiko, Kobe-shi, Hyogo 650-0047 (JP); SAKUMA Tetsushi, Hiroshima-shi, Hiroshima 739-8511 (JP); YAMAMOTO Takashi, Hiroshima-shi, Hiroshima 739-8511 (JP); NOMURA Wataru, Hiroshima-shi, Hiroshima 739-8511 (JP)
(74) Representative: Henderson, Helen Lee
(86) International application number: PCT/JP2024/021599
(87) International publication number: WO 2024/257843

(57) **Abstract**

The present invention provides a zinc-finger nuclease (ZFN) having three or more nuclear localization signals (NLSs) at the N-terminus as a technology that can be used to increase, particularly in non-dividing cells, the efficiency of genome editing using ZFNs.

## Description

### Technical Field

The present disclosure relates to a zinc-finger protein, a zinc-finger nuclease, a vector, and a genome editing method using them. The present disclosure relates more specifically to a zinc-finger protein and the like with three or more nuclear localization signals attached to the N-terminus.

### Background Art

The zinc-finger nuclease (ZFN) consists of a nuclease domain, namely FokI-ND, a nucleotide-binding domain, namely a zinc-finger protein (ZFP), and a linker that connects them. Patent Literature 1 discloses a ZFN containing a nuclease domain called nuclease domain 1 (ND1) or nuclease domain 2 (ND2) instead of FokI-ND. ND1 is the nuclease domain derived from *Bacillus* sp. SGD-V-76, and ND2 is the nuclease domain derived from *Clostridium botulinum.* ND1 and ND2 are considered to be superior to the conventional FokI-ND in terms of functionalities such as cleavage activity, specificity, and target sequence selectivity.

In relation to the present disclosure, Non-Patent Literature 1 reports that the addition of multiple nuclear localization signals (NLSs) to ZF-FokI-ND improves the genome editing efficiency in cultured cells (dividing cells). In addition, Non-Patent Literature 2 reports that the addition of one NLS to the N- and C-terminus of Cas nuclease enables efficient implementation of the Homology-independent Targeted Integration (HITI) method in non-dividing cells.

### Citation List

### Patent Literature

Patent Literature 1: WO 2020/045281

### Non Patent Literature

Non Patent Literature 1: "Improved Cell-Penetrating Zinc-Finger Nuclease Proteins forPrecision Genome Engineering Molecular Therapy", Jia Liu et al., Nucleic Acids, 2015, 4, e232
Non Patent Literature 2: "In vivo genome editing via CRISPR/Cas9 mediated homology-independent targeted integration", Keiichiro Suzuki et al., Nature, 2016, 540, 144-149
Non Patent Literature 3: "Standardized reagents and protocols for engineering zinc-finger nucleases by modular assembly", Wright et al., Nature Protocols, 2006, Vol. 1, No. 3, p. 1637-52.

### Summary of Invention

### Technical Problem

The main purpose of the present disclosure is to provide a technology that can be used to improve the efficiency of genome editing by ZFNs, especially in non-dividing cells.

### Solution to Problem

To solve the above problem, the present disclosure provides the following [1] to [13].
[1] A zinc-finger protein (ZFP) comprising three or more nuclear localization signals (NLSs) at an N-terminus thereof.
[2] The ZFP according to [1], wherein the NLSs are SV40 T antigen NLS(s) and/or c-myc NLS(s).
[3] The ZFP according to [2], comprising alternately arranged SV40 T antigen NLS and c-myc NLS.
[4] The ZFP according to [3], comprising two SV40 T antigen NLSs and one c-myc NLS.
[5] A vector comprising a nucleotide sequence encoding the ZFP according to any one of [1] to [4].
[6] A zinc-finger nuclease (ZFN) comprising the ZFP according to any one of [1] to [4] and a nuclease domain.
[7] The ZFN according to [6], wherein the nuclease domain is ND1.
[8] A ZEN according to [7], wherein the nuclease domain comprises
   an amino acid sequence at positions 391 to 585 of SEQ ID NO: 9,
   an amino acid sequence of SEQ ID NO: 14, or
   an amino acid sequence of SEQ ID NO: 15.
[9] A vector comprising a nucleotide sequence encoding any one of ZFNs according to [6] to [8].
[10] A method for genome editing, comprising, as a procedure, introducing the vector according to [9] into a cell.
[11] The genome editing method according to [10], wherein the method is implemented *in vitro.*
[12] The genome editing method according to [10] or [11], wherein the cell is a non-dividing cell.
[13] The method according to [12], wherein the non-dividing cell is a photoreceptor cell.

### Advantageous Effects of Invention

The present disclosure provides a technology that can be used to improve the efficiency of genome editing by ZFNs, especially in non-dividing cells.

### Brief Description of Drawings

[Fig. 1] Fig. 1 shows the upstream sequence of the exon 1 start codon of the mouse Rho gene and the recognition sequences of ZFPs (ZF-5057S and ZF5057AS) in ZF-ND1 expressed by the ZF-ND1 expression vector.
[Fig. 2] Fig. 2 shows the configurations of ZF-ND1 expression vectors (pRho2k-ZF-ND1DDD and pRho2k-ZF-ND1RRR).
[Fig. 3] Fig. 3 shows the donor gene vector (mRho-ZF-HITI-Donor).

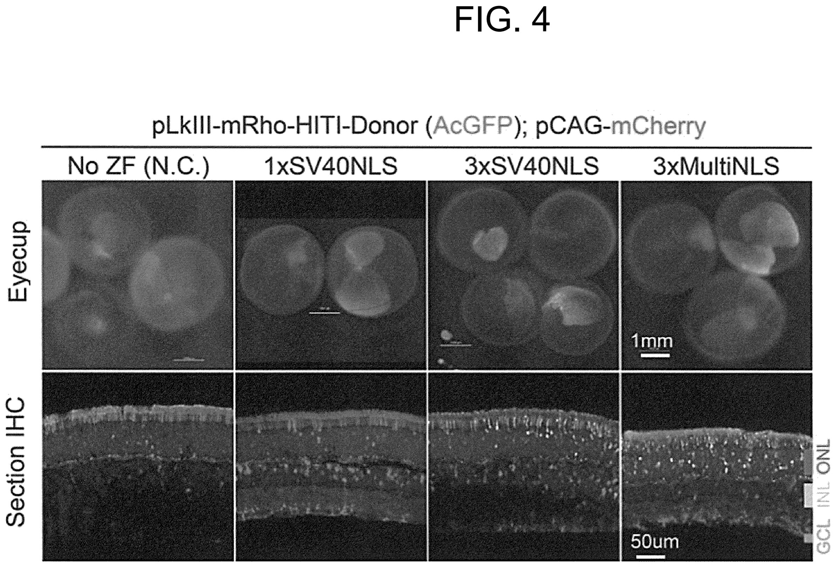

[Fig. 4] Fig. 4 shows the results of genome editing of rod photoreceptor cells by the Homology-independent Targeted Integration (HITI) method using ZF-ND1 expression vectors with three different nuclear migration signal sequences (1×SV40NLS, 3×SV40NLS, or 3×MultiNLS) inserted. The upper panel shows fluorescent images of retinal tissue (eyecup) recovered at P21 after introduction of a fluorescent protein (AcGFP) gene into the rhodopsin gene locus of rod photoreceptor cells in the mouse retina immediately after birth (P0-2). The lower panel shows fluorescent images of staining a retinal tissue with an anti-GFP antibody. The green indicates AcGFP, and the red indicates fluorescence of positive control mCherry.

### Description of Embodiments

### 1. Zinc-finger protein (ZFP)

The ZFPs of the present disclosure each contain a nucleotide binding domain and are characterized by having three or more nuclear localization signals (NLS) at the N-terminus of the nucleotide binding domain. The ZFPs of the present disclosure can exhibit improved genome editing efficiency by having three or more NLSs at the N-terminus, which are linked to the effector domain. The improved efficiency of genome editing can be achieved especially in non-dividing cells.

The nucleotide binding domain of each ZFP of the present disclosure comprises a zinc-finger array that recognizes a specific nucleotide sequence. The Zinc-finger array is composed of an array having multiple zinc-fingers (ZFs) each recognizing a three-nucleotide sequence. The zinc-finger array may contain two or more ZFs, e.g., 3-9, preferably 4-8, more preferably 5-7, or typically 6 ZFs. The nucleotide sequence recognized by the zinc-finger array can be set, if appropriate, according to the nucleotide sequence of the target nucleotide strand for genome editing and the nucleotide sequence of the editing target site in the target nucleotide strand. The relationship between the amino acid sequence of ZF and the three-nucleotide sequence recognized by ZF is well-known, and the amino acid sequence of ZF can be used, for example, as described in Supplemental Table 1 of Non-Patent Literature 1, depending on the three-nucleotide sequence to be recognized. Since there are multiple ZF amino acid sequences that recognize the same three-nucleotide sequence, these ZFs that recognize the same three-nucleotide sequence can be used interchangeably.

The NLSs possessed by each ZFP of the present disclosure may be 3 or more, preferably 3-5, and particularly 3 or 5.

As the NLSs possessed by each ZFP of the present disclosure, it is possible to use, without any particular limitation, the SV40 T antigen NLS (PKKKRKV: SEQ ID NO: 6), c-myc NLS (PAAKRVKLD: SEQ ID NO: 7), Nucleoplasmin NLS (KRXXXXXXXXXXXXKKKLD: SEQ ID NO: 8; X indicates any amino acid), and other conventionally known NLSs such as those NLSs having amino acid sequences derived from the above sequences.

The NLSs are preferably SV40 T antigen NLS(s) or c-myc NLS(s), and SV40 T antigen NLS(s) and c-myc NLS(s) are more preferred.

If the NLSs are SV40 T antigen NLSs and c-myc NLS, they may be arranged alternately, especially three NLSs are placed in the order of "SV40 T antigen NLS, c-myc NLS, and SV40 T antigen NLS".

Each NLS may be directly aligned or linked and aligned using a peptide linker. The amino acid sequence of the peptide linker is optionally provided, but may be, for example, AAA or GSG.

### 2. Zinc-finger nuclease (ZFN)

The effector domain to which the ZFP of the present disclosure is linked is not particularly limited, but may be, for example, a nuclease domain. Examples of another effector domain include Cytosine base editor (UGI, APOBEC), Adenine base editor (TadA), transcriptional activator (VP16)/repressor (SID), epigenomic editor (DNMT3A, TET1), imaging protein (Dye, fluorescent protein), transposase (PiggyBac), and recombinase (Flippase).

The effector domain may function alone (monomeric) or as a multimer (split-type).

The ZFP and effector domain may be linked directly or via a linker. The linker may, for example, comprise two or more amino acid residues and examples of the length include, but are not particularly limited to, 2 to 20 amino acids, e.g., 2, 3, 4, 5, 6, 7, 8, 9, 10, 11, 12, 13, 14, 14, 16, 17, 18, 19, or 20 amino acids. Examples of the kind of linker include, but are not particularly limited to, TGGS (SEQ ID NO: 13). The presence or absence of a linker and the length and kind of the linker are appropriately selected by a person skilled in the art while taking into consideration the type of effector domain and other factors.

The ZFP of the present disclosure is preferably linked to a nuclease domain to constitute a zinc-finger nuclease (ZFN). The ZFN of the present disclosure recognizes and binds to a specific nucleotide sequence by using a zinc-finger array, resulting in a DNA double-strand break (DSB) by the nuclease domain.

The nuclease domain is not particularly limited and may be FokI-ND, ND1, or ND2 described in Patent Literature 2, or their modified forms. The amino acid sequence of the full-length protein containing ND1 (from *Bacillus* sp. SGD-V-76) is set forth in SEQ ID NO: 59 and its nucleotide sequence in SEQ ID NO: 10. The amino acid sequence of the full-length protein containing ND2 (from *Clostridium botulinum*) is set forth in SEQ ID NO: 11 and its nucleotide sequence in SEQ ID NO: 12. ND1 is typically a partial peptide corresponding to positions 391-585 of SEQ ID NO: 9, and ND2 is typically a partial peptide corresponding to positions 389-579 of SEQ ID NO: 11. The nuclease domain should be ND1.

Modified forms of ND1 and ND2 may each contain an amino acid sequence in which one or several amino acid residues are substituted, deleted, inserted, or added in the amino acid sequence at positions 391 to 585 of SEQ ID NO: 9 or 389 to 579 of SEQ ID NO: 11, and may be a polypeptide having nuclease activity. Here, the "one or several" means, for example, 1 to 10, preferably 1 to 6, e.g., 1, 2, 3, 4, or 5.

Modified forms of ND1 and ND2 may contain an amino acid sequence having at least 70%, at least 75%, at least 80%, at least 85%, at least 90%, at least 95%, or at least 98% sequence identity to the amino acid sequence at positions 391 to 585 of SEQ ID NO: 9 or 389 to 579 of SEQ ID NO: 11, and may be a polypeptide having nuclease activity. Here, the "sequence identity" of an amino acid sequence is determined by comparing two sequences aligned such that the identity between sequences is maximum. How to calculate the sequence identity percentage (%) is well-known to those skilled in the art. As an algorithm to obtain the best alignment and sequence identity, any of the algorithms (e.g., BLAST algorithm, FASTA algorithm) known to those skilled in the art should be used. The sequence identity of an amino acid sequence is determined using sequence analysis software such as BLASTP or FASTA.

Specific examples of the modified forms of ND1 and ND2 include a modified form (DDD-type variant) having aspartic acid (D483, D487, and D496) at positions corresponding to positions 483, 487, and 496 in the amino acid sequence of FokI, or a modified form (RRR-type variant) having arginine (R483, R487, and R537) at positions corresponding to positions 483, 487, and 537 in the amino acid sequence of FokI. The DDD-type variant of ND1 (ND1DDD) has an amino acid sequence (SEQ ID NO: 14) in which amino acids 103 and 113 are replaced with aspartic acid in the partial peptide corresponding to positions 391-585 of SEQ ID NO: 9. In addition, the RRR-type variant of ND1 (ND1RRR) has an amino acid sequence (SEQ ID NO: 15) in which amino acids 100 and 154 are replaced with arginine in the partial peptide corresponding to positions 391-585 of SEQ ID NO: 9.

The nuclease domain of ZFN of the present disclosure may be a polypeptide containing modified and/or non-natural amino acids. Examples of the modified amino acid include, but are not limited to, methylation, esterification, amidation, acetylation, alkylation, and halogenation. The modified and non-natural amino acids can be introduced by known procedures.

The ZFP and ZFN of the present disclosure can be produced *in vitro* or *in vivo* by methods known in the art. Examples include an artificial synthesis method based on amino acid sequence information, or a method in which a nucleic acid encoding each ZFN is artificially synthesized, inserted into a suitable expression vector, and introduced in a suitable host cell to express the ZFNs in the cell.

### 3. Vector

The present disclosure also provides a vector containing a nucleotide sequence encoding the ZFP or ZFN described above.

In addition to the nucleotide sequence encoding ZFP or ZFN, the vector may contain elements provided by conventional gene expression vectors. Examples of such elements include promoters (e.g., the CMV promoter and the human EF1a (Elongation Factor alpha-1) promoter) and poly(A) addition sequences (e.g., SV40 p(A), HGH p(A), rabbit globin p(A)).

The vector is not particularly limited and can be selected from vectors used in the art. Specific examples of the vector include phage vectors, plasmid vectors, viral vectors, retroviral vectors, chromosomal vectors, episomal vectors, virus-derived vectors (e.g., bacterial plasmids, bacteriophages, yeast episomes), yeast chromosomal elements, viruses (e.g., baculovirus, papovavirus, vaccinia virus, adenovirus, adeno-associated virus, fowlpox virus, pseudorabies virus, herpesvirus, lentivirus, retrovirus), and vectors derived from combinations thereof (e.g., cosmids, phagemids). Among the above, plasmid vectors are preferred in terms of versatility. From the viewpoint of advanced clinical application, viral vectors are preferred, and adeno-associated virus vectors (AAV vectors) are more preferred.

### 4. Genome editing method

The present disclosure also provides a genome editing method that includes, as a procedure, introducing the above-described vector into a cell *in vivo* or *in vitro,* preferably *in vitro.*

Introduction of the vector into a cell can be performed by conventionally known methods. Examples of the introduction method include, but are not limited to, electroporation, particle gun, microinjection, lipofection, and protein transduction.

Depending on the purpose of genome editing, a donor DNA may be introduced into the cell together with the vector. The donor DNA may be, for example, a donor DNA encoding a normal gene. The donor DNA can be configured to introduce a normal gene into the gene locus by using a repair mechanism such as nonhomologous end joining (NHEJ) or homologous recombination (HR) at the cleavage site of DNA double-strand break by ZFNs.

In the case of genome editing without any donor DNA, the cleavage site is repaired mainly by NHEJ. Because NHEJ is error-prone, at least one nucleotide deletion, insertion, or substitution, or a combination thereof, can occur during repair of the cleavage. In this way, the target gene locus is modified at the cleavage site.

Cells may be of human or non-human animal origin. Examples of the non-human animals include even-toed ungulates (e.g., cattle, boars, pigs, sheep, goats), odd-toed ungulates (e.g., horses), rodents (e.g., mice, rats, hamsters, squirrels), hares (e.g., rabbits), and carnivores (e.g., dogs, cats, ferrets).

The cell may be either a dividing or non-dividing cell, but a non-dividing cell can be selected as particularly suitable in the present disclosure. Examples of the non-dividing cells include photocytes, lymphocytes, monocytes, neutrophils, eosinophils, basophils, endothelial cells, epithelial cells, hepatocytes, osteocytes, platelets, adipocytes, myocytes, neurons, retinal cells, smooth muscle cells, skeletal muscle cells, spermatocytes, oocytes, and pancreatic β cells.

### Examples

### [Example 1: Verification of effect of NLSs when foreign gene is inserted into non-dividing cells by HITI method using ZF-ND1]

The HITI (Homology-independent Targeted Integration) method is a technology to insert a foreign gene (donor gene) into the genome in a non-dividing cell with high efficiency and in a precise orientation. In this Example, the effectiveness of NLSs in the HITI method using ZF-ND1 as the genome editing factor was examined.

The following plasmid vectors were prepared and used for gene transfer into mouse rod photoreceptor cells (non-dividing cells) by *in vivo* electroporation.

### (1) ZF-ND1 expression vector

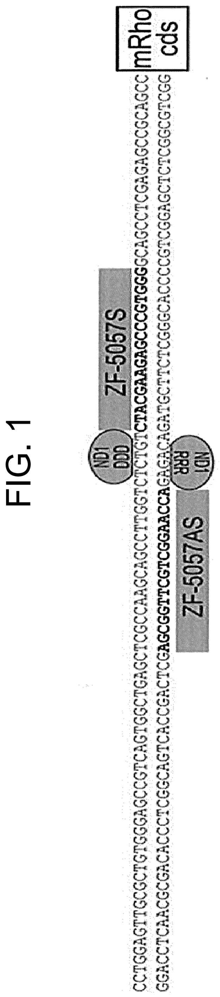

ND1 used for genomic DNA cleavage exhibits cleavage activity in a dimer form. Therefore, two ZFPs (paired ZFPs) corresponding to the sense and antisense strands were designed for the cleavage target sequence. Specifically, a ZFP pair was designed that recognizes each of the 18 nucleotide sequences located upstream of the exon 1 start codon of the mouse Rho gene. Fig. 1 shows the sequence upstream of the exon 1 start codon of the mouse Rho gene and the recognition sequences of the ZFP pair. A DDD-type variant of ND1 (ND1DDD, SEQ ID NO: 14) was linked to a sense strand ZFP (ZF-5057 S), which has a zinc-finger array that recognizes and binds to the sense strand sequence CTACGAAGAGCCCGTGGG (SEQ ID NO: 1), to form ZF-5057S-ND1DDD. The amino acid sequence of ZF-5057S-ND1DDD is shown in SEQ ID NO: 16. The RRR-type variant of ND1 (ND1RRR, SEQ ID NO: 15) was linked to the antisense strand ZFP (ZF-5057AS), which has a zinc-finger array that recognizes and binds to the antisense strand sequence ACCAAGGCT GCTTGGCGA (SEQ ID NO: 2), to form ZF-5057AS-ND1RRR. The amino acid sequence of ZF-5057AS-ND1RRR is shown in SEQ ID NO: 17. A peptide linker (sequence Thr-Gl y-Gly-Ser: SEQ ID NO: 13) was used for the linkage between ND1 and ZFP.

The nucleotide sequence encoding ZF-5057AS-ND1RRR or ZF-5057S-ND1DDD was incorporated into a plasmid vector to construct the expression vector pRho2k-ZF-ND1DDD or pRho2k-ZF-ND1RRR, respectively.

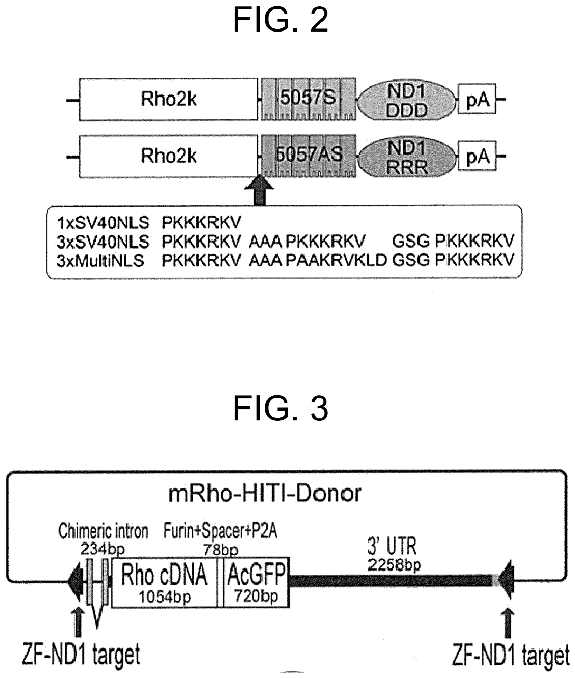

The bovine Rho promoter (2174 bp) was used as the promoter, and rabbit beta-globin poly(A) was used as the poly(A) addition sequence. Fig. 2 shows the structure of each expression vector. For each expression vector, one of the following three nuclear localization signal sequences was inserted at the N-terminus of ZF.
A) SV40 NLS 1 sequence (1×SV40NLS): PKKKRKV (SEQ ID NO: 3);
B) SV40 NLS 3 sequence (3×SV40NLS): PKKKRKV AAA PKKKRKV GSG PKKKRKV (SEQ ID NO: 4); and
C) SV40 NLS, c-Myc NLS, and SV40 NLS in this order (3×MultiNLS): PKKKRKV AAA PAA KRVKLD GSG PKKKRKV (SEQ ID NO: 5)
(The underlined amino acid sequence indicates an NLS sequence; AAA or GSG indicates a linker sequence).

### (2) Donor gene vector

The donor gene was a cDNA of the mouse Rho gene to which a nucleotide sequence encoding a fluorescent protein GFP was added. A construct, in which a chimeric intron sequence, mouse rhodopsin cDNA (Rho cDNA), a self-cleaving peptide sequence (Furin+Spacer+P2A), AcGFP, and a 3'UTR sequence from the mouse Rho gene locus were linked in this order, was introduced into a pLeaklessIII plasmid. In addition, a sequence (ZF-ND1 target), in which the ZF recognition sequence was placed in an opposite orientation, was inserted at both ends of this construct. The resulting gene cassette was named mRho-ZF-HITI-Donor (see Fig. 3).

### (3) mCherry expression vector

A plasmid was constructed to express mCherry red fluorescent protein under the control of the CAG promoter.

Here, 0.3-0.4 µL of vector solution adjusted to a concentration of 5-7 µg/µL was injected under the mouse retina immediately after birth (P0-2). Gene transfer was performed by applying electric pulses using tweezer-type electrodes (Nepagene CUY650-7), with the head held between the electrodes so that the retinal side was set as the positive electrode side. The mixing ratio of the vector solutions was as follows in percentage.
pRho2k-ZF-ND1DDD 15%
pRho2k-ZF-ND1RRR 15%
mRho-ZF-HITI-Donor 60%
pCAG-mCherry 10%

The Rho promoter in pRho2k-ZF-ND1DDD and pRho2k-ZF-ND1RRR becomes active from around P7 to P10 after the differentiation of rod photoreceptor cells. Accordingly, the expression of ZF-ND1DDD and ZF-ND1RRR begins in rod photoreceptor cells after differentiation (that is, after the cessation of cell division). Eyes were collected at P21, when cell differentiation of the retina was largely complete. The outer scleral portion was peeled off and removed, and the remaining tissue (eyecup) was fixed with 4% paraformaldehyde solution (Nacalai). Fluorescent images of GFP and mCherry were then captured using a fluorescence stereomicroscope. Tissue sections were also prepared and subjected to fluorescent immunostaining with an anti-GFP antibody.

Fluorescent images of eyecups with pRho2k-ZF-ND1DDD and pRho2k-ZF-ND1RRR with three different nuclear localization signal sequences (1×SV40NLS, 3×SV40NLS, or 3×MultiNLS) are shown in the upper panel of Fig. 4 (green shows AcGFP and red shows mCherry fluorescence).

The construct with one NLS sequence (1×SV40NLS) showed almost no green fluorescence and was comparable to the negative control group without ZF-ND1 vector (No ZF). In the constructs with three NLS sequences, green fluorescence was increased in the order of 3×SV40NLS and 3×Multi40NLS.

Fluorescent images obtained by staining with an anti-GFP antibody in the case of using pRho2k-ZF-ND1DDD and pRho2k-ZF-ND1RRR with three different nuclear localization signal sequences (1×SV40NLS, 3×SV40NLS, or 3×MultiNLS) are shown in the lower panel of Fig. 4.

AcGFP-positive cells were observed only in the outer nuclear layer (ONL), where photoreceptor cells are located, whereas mCherry-positive cells were also observed in the inner nuclear layer (INL), where horizontal cells, bipolar cells, and amacrine cells are located. This confirms that knock-in of the mRho-ZF-HITI-Donor cassette occurred in a photoreceptor-limited manner. AcGFP-positive photoreceptor cells were increased in the order of 1×SV40NLS, 3×SV40NLS, and 3×Multi40NLS.

These results indicate that efficient genome editing in photoreceptor cells (non-dividing cells) is possible by adding multiple (three) NLSs to ZFP.

### Sequence listing free text

SEQ ID NO: 1: recognition sequence of ZF-5057S
SEQ ID NO: 2: recognition sequence of ZF-5057AS
SEQ ID NO: 3: amino acid sequence of SV40 NLS 1 sequence (1×SV40NLS)
SEQ ID NO: 4: amino acid sequence of SV40 NLS 3 sequence (3×SV40NLS)
SEQ ID NO: 5: amino acid sequence of SV40 NLS, c-Myc NLS, and SV40 NLS in that order (3×MultiNLS)
SEQ ID NO: 6: amino acid sequence of SV40 T antigen NLS
SEQ ID NO: 7: amino acid sequence of c-myc NLS
SEQ ID NO: 8: amino acid sequence of Nucleoplasmin NLS
SEQ ID NO: 9: amino acid sequence of the full-length protein (from *Bacillus* sp. SGD-V-76) containing ND1
SEQ ID NO: 10: nucleotide sequence of the full-length protein (from *Bacillus* sp. SGD-V-76) containing ND1
SEQ ID NO: 11: amino acid sequence of the full-length protein (from *Clostridium botulinum*) containing ND2
SEQ ID NO: 12: nucleotide sequence of the amino acid sequence of the full-length protein (from *Clostridium botulinum*) containing ND2
SEQ ID NO: 13: amino acid sequence of a peptide linker
SEQ ID NO: 14: amino acid sequence of ND1DDD
SEQ ID NO: 15: amino acid sequence of ND1RRR
SEQ ID NO: 16: amino acid sequence of ZF-5057S-ND1DDD
SEQ ID NO: 17: amino acid sequence of ZF-5057S-ND1RRR

## Claims

1. A zinc-finger protein (ZFP) comprising three or more nuclear localization signals (NLSs) at an N-terminus thereof.

2. The ZFP according to claim 1, wherein the NLSs are SV40 T antigen NLS(s) and/or c-myc NLS(s).

3. The ZFP according to claim 2, comprising alternately arranged SV40 T antigen NLS and c-myc NLS.

4. The ZFP according to claim 3, comprising two SV40 T antigen NLSs and one c-myc NLS.

5. A vector comprising a nucleotide sequence encoding the ZFP according to any one of claims 1 to 4.

6. A zinc-finger nuclease (ZFN) comprising the ZFP according to any one of claims 1 to 4 and a nuclease domain.

7. The ZFN according to claim 6, wherein the nuclease domain is ND1.

8. A vector comprising a nucleotide sequence encoding the ZFN according to claim 6.

9. A method for genome editing, comprising, as a procedure, introducing the vector according to claim 8 into a cell.

10. The method according to claim 9, wherein the cell is a non-dividing cell.

11. The method according to claim 10, wherein the non-dividing cell is a photoreceptor cell.
